# EUROPEAN PATENT SPECIFICATION

(11) **EP 0 580 453 B1**
(45) Date of publication and mention of the grant of the patent: **08.03.2000**
(21) Application number: 93305858.8
(22) Date of filing: 23.07.1993
(51) Int. Cl.: A61K 7/06

(54) **Use of N-alkoxyalkylamides in hair-care compositions**
Verwendung von N-Alkoxyalkylaminen in Haarpflegemitteln
Utilisation de N-alkoxyalkoylamines dans des compositions pour le soin des cheveux

(30) Priority: 24.07.1992 US 920220
(43) Date of publication of application: 26.01.1994
(73) Proprietor: Revlon Consumer Products Corporation, New York, NY 10022 (US)
(72) Inventor: Goldberg, Marvin Eugene, Marlboro, New Jersey 07746 (US); Bhambhani, Malti Vishin, Scotch Plains, New Jersey 07076 (US); Brandon, Arthur, Valley Cottage, New York 10989 (US)
(74) Representative: Sanderson, Laurence Andrew

(56) References cited:
- EP-A- 0 338 565

## Description

This invention relates to the use of one or more N-alkoxyalkylamide(s) in the manufacture of hair-care compositions, notably compositions such as shampoos, hair-conditioners, hair-sprays, and hair-setting and hair-styling preparations.

Hair-shampoos as their most fundamental ingredient usually contain anionic detergents, which foam and clean the hair - but while very effective in their intended cleansing function such anionic detergents can be harsh in their effect upon the hair surface, leaving it in an unduly dry, unmanageable state. Furthermore, hair thus cleansed is rendered thereby susceptible to the accumulation of electrostatic charges, which give rise to an undesirable condition known as "fly-away" hair.

In order to combat these adverse effects, it is known and indeed usual practice to incorporate other ingredients into the shampoo formulation (besides those e.g. perfume that contribute to product aesthetics) which serve to moderate the ill-effects of anionic detergents - but while these can be effective in providing greater manageability and hair-conditioning, they unfortunately interfere with and reduce the effectiveness of the anionic detergent in cleansing and foaming.

On the other hand hair-conditioners and creme-rinse preparations employ cationic surfactants, usually quaternary ammonium compounds, as their key ingredients. These materials are substantive to the negatively-charged keratinaceous protein of hair, and consequently treatment of hair with a hair-conditioner containing cationic quaternary ammonium compounds (generally after shampooing) does serve to provide the hair with a lubricating surface film which allows for easy combing. Thus knots and tangles, encountered when combing hair after shampooing, are easily dissipated through hair-treatment with a cationic hair-conditioner. Nevertheless, despite their desirable characteristics, such cationic hair-conditioners formulated with quaternary ammonium compounds tend to over-condition the hair, making it undesirably heavy, "bodiless" and unattractive in appearance.

As regards hair-setting lotions and hair-sprays, these are usually based upon polymeric resins - which provide a hair-fixative effect. Hair gains rigidity through application of these products, enabling it to hold in place whatever particular style-configuration has been imparted to it. However, it is usually thought necessary also to incorporate plasticizers into these hair-setting lotions and hair-spray products, in order to impart pliability to the film of the polymeric resin that coats the hair, since the presence of plasticizers allows the resin film to bend, Instead of rupturing or breaking under the stress of slight hair-movement. The overall hair-holding ability of the polymeric hair-fixative is however reduced when plasticizers are incorporated, and as a result the strength of the hair configuration is weakened, and the hair styling becomes short-lived.

We have now found that a small amount of certain selected N-alkoxylalkylamides (already known to be useful for other, unrelated purposes - see our earlier EP-A-0,338,565) when incorporated with the key ingredients of an anionic shampoo, cationic hair-conditioner or resinous hair-fixative product, will serve to impart an unique feel, lubricity and sheen to hair treated with this product, without causing it to be greasy or heavy. In addition, hair thus treated is smoother, easier to manage, and has increased body, fullness and softness. Moreover the selected N-alkoxyalkylamides used according to the present invention appear to be fully compatible in the formulations with the various anionic surfactants, cationic surfactants and polymeric resins used in the respective formulation environments.

According to this invention there is provided the use, in the manufacture of hair-care compositions that improve the condition of the hair of (besides other ingredients appropriate to their envisaged use), of one or more N-alkoxyalkylamide(s) of the general formula: 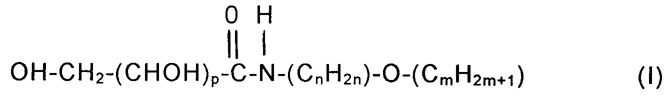
wherein p is a positive integer of from 1 to 4,
(CₙH₂ₙ) is a straight- or branched-chain alkylene linkage in which n is a positive integer of from 1 to 6; and
(CₘH₂ₘ₊₁) is a straight- or branched-chain alkyl radical in which m is a positive integer of from 1 to 6.

The hair-care compositions made in accordance with this invention are preferably those which contain one or more compound(s) of the general formula I above wherein p is a positive integer from 1 to 4, (CₙH₂ₙ) is a straight- or branched-chain alkylene linkage in which n is a positive integer of from 1 to 4, preferably 3, and (CₘH₂ₘ₊₁) is a straight- or branched-chain alkyl radical in which m is a positive integer of from 1 to 4, preferably 1.

Above all we prefer to employ the N-alkoxyalkylamide(s) of general formula I wherein p is 4, n is 3, and m is 1.. and specifically N-methoxy-propyl-gluconamide (hereinafter abbreviated to "MPG") having the structural formula:

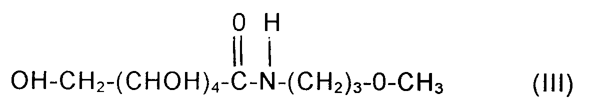

While the N-alkoxyalkylamide(s) of general formula I or the specifically preferred such compound MPG of structural formula III can be used outside the following limits, we prefer to formulate the composition so as to contain from 0.001 to 10% thereof, especially from 0.005 to 7%, and above all from 0.01 to 5% thereof.

It will however be understood that the amount of the N-alkoxyalkylamide(s) I (and II) in any particular case and the nature and amount of the other ingredient(s) is liable to vary according to the intended use of the compositions.

The hair-care compositions made according to this invention can be intended for several different purposes, and thus they can be for instance shampoos, hair-sprays, gels, conditioners, mousses or the like.

When the N-aloxyalkylamide(s) I/II is/are incorporated into shampoos, the resulting shampoo imparts excellent lubricity, sheen and manageability to hair, without giving it a greasy or heavy feeling. The hair is also smoother and easier to manage, and has improved body, fullness and softness. Generally-speaking, suitable shampoo formulations will contain from 1 to 45% of anionic surfactant(s), and from 1 to 55% of water. Such shampoos may and usually will additionally contain foam boosters and/or thickeners and/or chelating agents and/or preservatives

Foam-boosters are generally added at a concentration of from 0.01 to 10%. Suitable foam boosters include cocamide MEA, cocamide DEA, lauramide DEA, capramide DEA, cetearyl alcohol, cetyl alcohol, cetyl betaine, cocamide MIPA, cocamidoethyl betaine, cocamidopropylamine oxide, cocamidopropyl betaine, cocamidopropyl hydroxysultaine, cocamide oxide, coconut alcohol, coco-sultaine, lauryl betaine, lauryl sultaine, lauryl alcohol, myristamide DEA, myristamide MEA, oleyl betaine, sodium sarcosinates and postassium coco hydrolyzed silk protein.

Thickeners of many kinds may be employed, usually in a concentration range of from 0.01 to 10%, and suitable ones include cellulose derivatives such as hydroxyethyl cellulose, hydroxypropyl cellulose, C₉₋₁₅ alcohols, Carbomers, guar gum and magnesium silicate, as well as various acrylic acid derivatives, PEG monostearates, etc.

Preservative(s) should generally be included, usually in a concentration of from 0.001 to 1%, and the preservative(s) suitably employed is/are those which are traditionally used in shampoo or other hair-care compositions.

Chelating agent(s) may also preferably be incorporated in shampoo compositions, which is/are capable of complexing with and thereby inactivating metal ions which otherwise might exert adverse effects on the formulation. Such chelating agent(s) should generally be present in a concentration of from 0.001 to 1%, and suitable chelating agents are for instance EDTA, HEDTA, TEA-EDTA, sodium metasilicate, sodium metaphosphate and sodium citrate, or mixtures thereof.

When the N-alkoxyalkylamide(s) of general formula I are incorporated in hair-conditioning compositions they impart excellent properties to hair when present in a suitable concentration of from 0.001 to 10%, preferably from 0.005 to 7%, and above all of from 0.01 to 5%.

Such conditioning compositions will also generally contain from 1.0 to 10% of a cationic polymer, from 10 to 95% of water, and from 0.1 to 10% of a conditioning agent.

Suitable cationic polymers include cationic quaternary ammonium compounds such as cetyl trimethyl ammonium chloride, cetrimonium chloride, stearylamidopropyl ethyldimonium ethosulfate, Polyquat 11, oleylamidopropyl amine oxide, guar hydroxypropyl trimonium chloride, trimethylsilylamodimethicone, stearyl dimethyl benzyl ammonium chloride, cetyl pyridinium chloride and dicetyl dimethyl ammonium chloride, or mixtures thereof.

Suitable conditioning agents include cetyl alcohol, hydrolyzed collagen, stearamidopropyl dimethyl amine, wheat germ oil, silk protein, keratin amino acids, cocdimonium silk amino acids, panthenol, dimethicone and dimethicone copolyol, or mixtures thereof.

The conditioning agents may also contain emulsifiers, thickeners, acidulants, preservatives and fragrances, etc.

Emulsifiers are generally desirable in a concentration of from 0.01 to 5%, and suitable emulsifiers include ethoxylated cetyl/stearyl alcohol and cetearyl alcohol.

Thickeners are also generally desirable in a concentration of from 0.01 to 5%, and suitable thickeners include cellulose derivatives and PPG-Buteth derivatives.

Acidulants may also need to be added so as to keep the pH of the conditioner formulation in the desired range of from 2.5 to 6.0; and when acidulants such as citric acid and the like are employed for that purpose they are generally added in a concentration range of from 0.001 to 1%.

When the N-alkoxyalkylamide(s) is/are used in hair-sprays or hair-fixative compositions, again they impart sheen, lubricity and softness to the hair. These compounds are to be added to such compositions in amounts of from 0.001 to 10%, preferably from 0.005 to 7%, and above all from 0.005 to 5%.

A suitable hair-spray composition comprises 0.5-10% resin, 0.001-5% resins, 0.01-5% of neutralizers, 0.01-5% of plasticizers, 15-40% of a solvent and, if desired, 5-60% of propellant.

Suitable resins include vinyl acetate / crotonic acid / vinyl neodecanoate copolymer, octyl acrylamide / acrylates / butyl amino ethyl methacrylate copolymer, vinyl acetate / crotonic acid, polyvinylpyrrolidone, polyvinyl pyrrolidone vinyl acetate copolymer, PVP acrylates copolymer, the ethyl ester of polyvinyl methylether maleic anhydride, polyvinylpyrrolidone acetate, polymeric quaternary ammonium salts prepared by the reaction of ethyl methacrylate / abietylmethacrylate / diethylamino ethyl methacrylate copolymer with dimethyl sulfate and a polymeric quaternary ammonium salt prepared by the reaction of ethyl methacrylate / oleylmethacrylate / diethylamino ethyl methacrylate copolymer with dimethyl sulfate. Suitable neutralizers include amino methyl propanol, etc. Suitable plasticizers include ethyoxylated lanolin alcohols, C₁₂₋₁₅ alcohols, benzoate, glycerine, etc. Suitable solvents include isopropyl alcohol and anhydrous ethanol, or mixtures thereof.

The compounds of general formula I and structural formula II may also be added to other hair-fixative compositions such as aerosol hair-sprays, hair-setting lotions and styling aids. Suitable hair-setting lotions contain from 0.01 to 10% of hair-fixative, from 0.01 to 5% of plasticizer and from 20 to 70% solvent, or mixtures thereof.

Suitable hair-fixatives include polyvinylpyrrolidone (PVP) vinyl acetate, polyquaternium compounds and polyvinylpyrrolidone, or mixtures thereof. Suitable plasticizers include glycerine, ethyoxylated lanolin alcohols, C₁₂₋₁₅ alcohols, benzoate. Suitable solvents include methylene chloride, isopropyl alcohol and anhydrous ethanol, or mixtures thereof.

The compounds of general formula I and structural formula II may also be incorporated into mousse compositions. Suitable mousse compositions contain from 0.1 to 10% of hair-fixative, from 0.1 to 5% of conditioner, from 0.05 to 5% of foaming agent, and from 1 to 50% of solvent.

The hair-fixatives, conditioners and foaming agents are as mentioned herein. Suitable foaming agents include octoxynol.

In order that the invention shall be better understood it will now be further described in connection with the following Examples, which however are provided for purposes of illustration only:

### Example 1

A hair-spritzer was made according to the following formula:

| | w/w% |
|---|---|
| SD alcohol 40 B | 80.00 |
| AMP | 0.71 |
| Resin 28-2930 | 5.00 |
| Amphomer | 1.5 |
| Dimethicone copolyol | 0.30 |
| Silk Protein | 0.10 |
| Cocamidopropyl Betaine | 0.10 |
| Water | QS |
| Fragrance | 0.30 |
| Methoxypropylgluconamide | 0.10 |
| Benzophenone 3 | 0.30 |

### Example 2

A hair-styling gel was made as follows:

| | w/w% |
|---|---|
| Water | 74.21 |
| Carbomer 940 | 17.00 |
| Water | 2.00 |
| PVPK-30 | 4.80 |
| Triethanolamine | 0.60 |
| Kathon CG | 0.04 |
| DMDM Hydantoin | 0.40 |
| PEG-75 Lanolin | 0.50 |
| PEG-75 Lanolin | 0.50 |
| Fragrance | 0.03 |
| Methyl paraben | 0.10 |
| PEG 40 hydrogenated Castor Oil | 0.30 |
| Hydrolyzed Silk | 0.10 |
| Panthenol | 0.10 |
| Benzophenone 4 | 0.05 |
| Methoxypropylgluconamide | 0.10 |

### Example 3

A shampoo for dry and damaged hair was made as follows:

| | w/w% |
|---|---|
| Water | qs |
| Sodium Lauryl Ether Sulfate | 14.00 |
| Decyl Polyglucose | 14.00 |
| Lauramide DEA | 3.00 |
| Citric Acid | 0.05 |
| Methylparaben | 0.10 |
| Propylparaben | 0.05 |
| Guar Hydroxypropyltrimonium Chloride | 0.30 |
| Water | 10.00 |
| Glycol Stearate | 1.70 |
| Myristic Acid | 0.20 |
| Sodium Lauryl Ether Sulfate | 10.00 |
| Dimethicone Copolyol | 0.20 |
| Glycerin | 0.20 |
| Trisodium HEDTA | 0.23 |
| Methoxypropylgluconamide | 0.25 |
| Kathon CG | 0.04 |

### Example 4

A hair-fixative gel was made as follows:

| | w/w% |
|---|---|
| Water | qs |
| Polyvinylpyrrolidone | 4.00 |
| Hydrolyzed Silk Protein | 0.10 |
| Water | 35.00 |
| Propylene Glycol | 0.30 |
| Methylparaben | 0.10 |
| Polyquat 11 | 1.00 |
| Polysorbate 20 | 0.50 |
| Kathon CG | 0.04 |
| Fragrance | 0.30 |
| Methoxypropylgluconamide | 0.0001 |

### Example 5

A hair-conditioner was made as follows:

| | w/w% |
|---|---|
| Water | 72.18 |
| Polyvinylpyrrolidone | 1.00 |
| Propylene glycol | 2.00 |
| Dicetylcimonium chloride | 1.00 |
| Octylmethoxy cinnamate | 0.01 |
| Hydroxyethyl cellulose | 1.25 |
| Fragrance | 0.60 |
| Water | 20.00 |
| Cetyl alcohol | 0.50 |
| Stearamido propyl stearamine | 0.50 |
| Ceatearyl alcohol/ceteareth 20 | 2.00 |
| Propyl paraben | 0.02 |
| Methyl paraben | 0.10 |
| Methoxypropyl gluconamide (50%) | 0.25 |
| Hydrolyzed Silk | 0.10 |
| Kathon CG | 0.04 |

### Example 6

An aerosol hair-spray was prepared according to the following formula:

| | w/w% |
|---|---|
| SD 40B alcohol | QS |
| AMP | 0.60 |
| Vinyl acetate crotonic acid polymer vinyl neodecanoate | 5.000 |
| Polysorbate 80 | 0.200 |
| Polysorbate 20, acetylated alcohol | 0.100 |
| PPG-20 Methyl glucose ether | 0.070 |
| Fragrance | 0.200 |
| Ethyl ester of hydrolyzed silk | 0.100 |
| Methoxypropyl gluconamide | 0.050 |

About 70 parts of the above mixture was mixed with about 17 parts Dymel 152A and 13 parts of Hydrocarbon A17.

### Example 7

An aerosol hair-spray was made as follows:

| | w/w% |
|---|---|
| SD 40B alcohol | QS |
| AMP | 0.65 |
| Vinyl acetate/crotonic acid vinyl neodecanoate copolymer | 7.00 |
| Fragrance | 0.20 |
| Ethyl ester of hydrolyzed silk | 0.10 |
| Water | 25.00 |
| Cyclomethicone | 0.10 |
| Methoxypropylgluconamide | 0.05 |

About 70 parts of the above formulation was mixed with 10 parts of Hydrocarbon A31 and 20 parts of Dymel A.

### Example 8

A shaving foam was made as follows:

| | w/w% |
|---|---|
| Water | QS |
| Methylparaben | 0.20 |
| Glycerin | 8.00 |
| Allantoin | 0.25 |
| PVP | 0.25 |
| Dioctyl sodium sulfosuccinate | 1.00 |
| TEA (98%) | 3.70 |
| Palmitic acid | 6.00 |
| Stearic acid | 2.00 |
| Propylparaben | 0.10 |
| Lanolin oil | 0.30 |
| BHT | 0.01 |
| Jojoba oil | 1.50 |
| Water | 2.00 |
| Fragrance | 1.00 |
| Methoxypropylgluconamide | 0.50 |

About 96 parts of the above formulation was mixed with 4 parts of hydrocarbon propellant.

### Example 9

A shaving gel was made as follows:

| | w/w% |
|---|---|
| Water | QS |
| FD&C Blue #1 | QS |
| Hydroxypropylcellulose | 0.15 |
| Sorbitol | 6.00 |
| Triethanolamine (98%) | 8.00 |
| Palmitic acid | 10.00 |
| Propylene glycol isostearate | 3.00 |
| Fragrance | 0.50 |
| Methoxypropylgluconamide | 0.50 |

About 96 parts of the above formulation was mixed with 4 parts of hydrocarbon propellant.

### Example 10

A brushless shave cream was made as follows:

| | w/w% |
|---|---|
| Water | QS |
| Polyquaternium 10 | 0.05 |
| PEG 14M | 0.20 |
| Butylene glycol | 2.50 |
| Glycerin | 3.00 |
| Cocamido propyl betaine | 4.00 |
| Methyl paraben | 0.35 |
| Propyl paraben | 0.20 |
| Stearic acid | 16.00 |
| Glycol stearate | 2.00 |
| Phenyl trimethicone | 2.50 |
| Dimethicone | 1.00 |
| Sorbitan laurate | 0.50 |
| Polysorbate 20 | 2.00 |
| Lanolin, isopropyl lanolate | 7.00 |
| TEA (98%) | 1.60 |
| Fragrance | 0.50 |
| Methoxypropylgluconamide | 0.50 |

### Evaluation of the effectiveness of the N-Alkoxyalkylamides

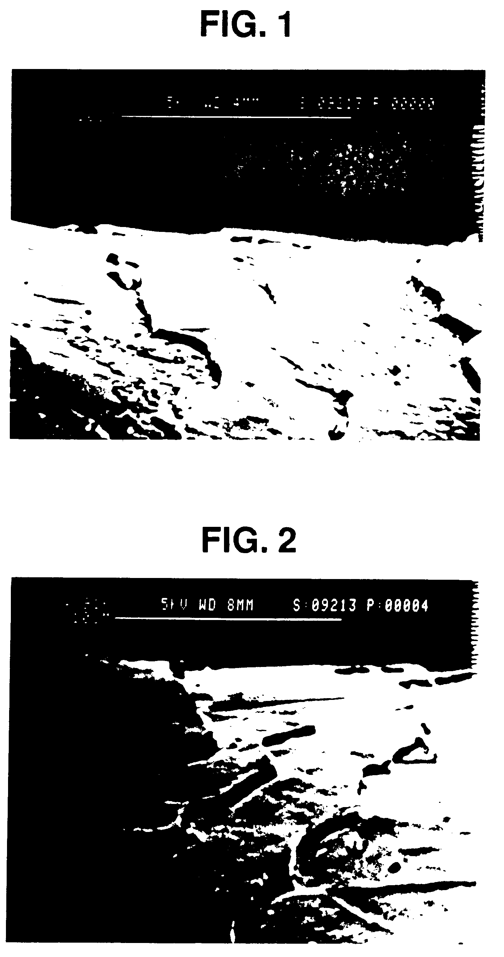

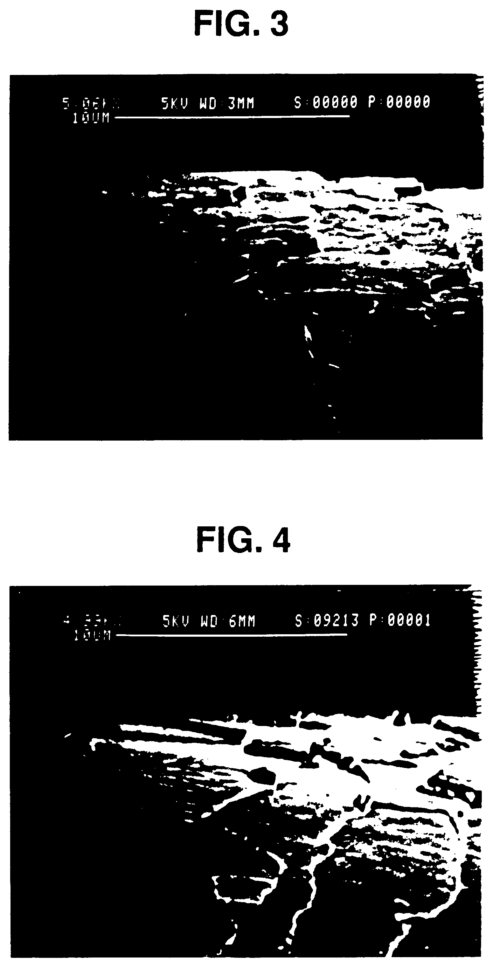

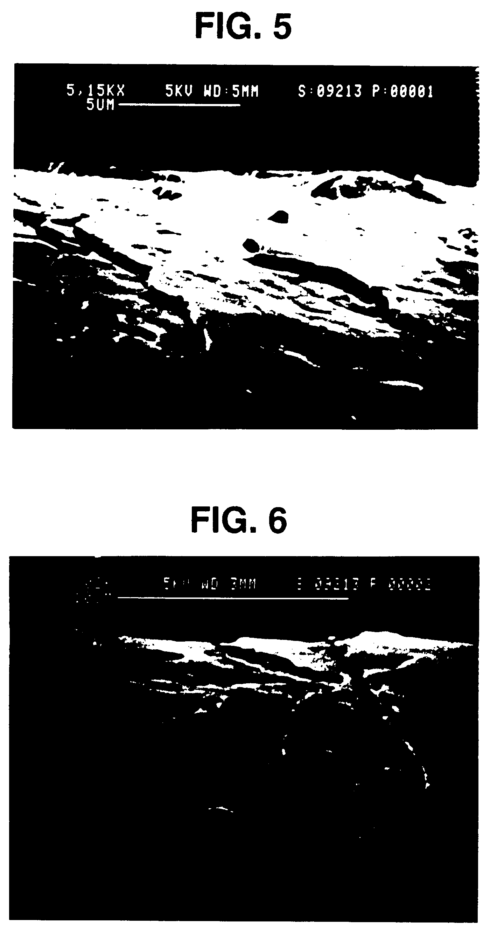

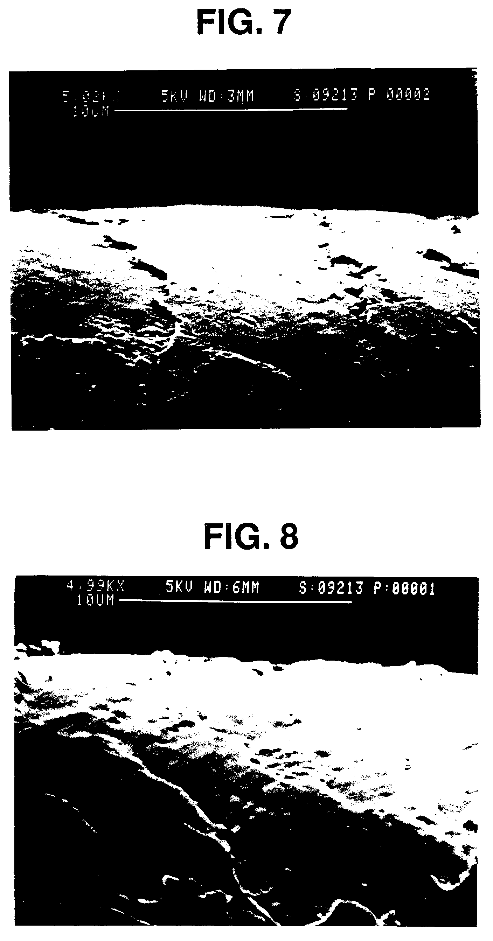

Brown and bleached hair tresses were washed, either once or ten times, with either a conventional shampoo composition or with a shampoo composition to which 5% N-methoxypropylgluconamide had been added and thus in accordance with this invention. Then the tresses were observed under the microscope at amagnification of 5000X; the photomicrographs thus obtained appear as Figures 1-8 of the accompanying drawings.

In the drawings:
Figure 1 is a photograph at 5000X magnification of a brown hair tress which had been shampooed ten times with a standard shampoo composition also containing 5% of N-methoxypropylgluconamide. The brown hair tress shows build-up of material on and between the cuticle layers of the tress.
Figure 2 is a photograph at 5000X magnification of a brown hair tress which had been shampooed ten times with a standard shampoo composition that did not contain N-methoxypropylgluconamide. The tress does not show any build-up of material on and between the cuticle layers such as is seen on the tress of Figure 1.
Figure 3 is a photograph at 5000X magnification of a brown hair tress which had been shampooed once with a standard shampoo composition also containing 5% of N-methoxypropylgluconamide. A slight build-up of material on and between the hair cuticle is seen.
Figure 4 is a photograph at 5000X magnification of a brown hair tress which had been shampooed once with a standard shampoo composition that did not contain N-methoxypropylgluconamide. No build-up of material is seen on the cuticle.
Figure 5 is a photograph at 5000X magnification of a bleached hair tress which had been shampooed ten times with a standard shampoo composition also containing 5% of N-methoxypropylgluconamide. A significant build-up of material on and between the cuticles is evident.
Figure 6 is a photograph at 5000X magnification of a bleached hair tress which had been shampooed ten times with a standard shampoo composition that did not contain methoxypropylgluconamide. In contrast to Figure 5, no build-up on or between the cuticles is to be seen.
Figure 7 is a photograph at 5000X magnification of a bleached hair tress which had been shampooed once with a standard shampoo composition also containing 5% of N-methoxypropylgluconamide. A slight build-up of material is seen on and between the cuticles.
Figure 8 is a photograph at 5000X magnification of a bleached hair tress which had been shampooed once with a standard shampoo composition that did not contain N-methoxypropylgluconamide. In contrast to Figure 7, no build-up of material is seen on or between the cuticles.

The results thus obtained can be summarized as follows. The tresses shampooed ten times with shampoo containing 5% of N-methoxypropylgluconamide exhibited a significant build-up of material on the hair cuticle, as compared to the hair tresses shampooed ten times with the same shampoo composition but without N-methoxypropylgluconamide. Even the bleached and brown hair tresses which were shampooed only once with shampoo containing 5% of N-methoxypropylgluconamide exhibited some build-up of material, which was not to be seen in the hair tresses shampooed with the same shampoo composition but without N-methoxypropylgluconamide.

## Claims

1. Use, in the manufacture of hair-care compositions that improve the condition of the hair, of one or more N-alkoxyalkylamide(s) of the general formula: 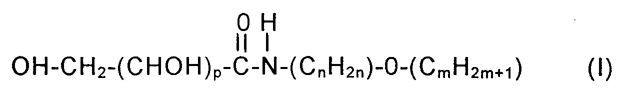
wherein p is a positive integer from 1 to 4,
(CₙH₂ₙ) is a straight- or branched-chain alkylene linkage in which n is a positive integer of from 1 to 6, and
(CₘH₂ₘ₊₁) is a straight- or branched-chain alkyl radical in which m is a positive integer of from 1 to 6.

2. Use as claimed in claim 1 in which the N-alkoxyalkylamide is or includes one wherein p is from 1 to 4, n is from 1 to 4 and m is from 1 to 4.

3. Use as claimed in claim 1 or claim 2, in which the N-alkoxyalkylamide is or includes one in which p is 4, n is 3 and m is 1.

4. Use as claimed in any of the preceding claims, in which the N-alkoxyalkylamide is or includes N-methoxy-propyl-gluconamide.

5. Use as claimed in any of the preceding claims, in which the composition is formulated to contain from 0.001 to 10% of the N-alkoxyalkylamide(s).

6. Use as claimed in any of the preceding claims, in which the composition is formulated to contain from 0.005 to 7% of the N-alkoxyalkylamide(s).

7. Use as claimed in any of the preceding claims, in which the composition is formulated to contain from 0.01 to 5% of N-methoxypropyl-gluconamide.

8. Use as claimed in any of the preceding claims, in which the hair-care composition is formulated as a shampoo, conditioner, hair-spray, mousse or gel.

9. Use as claimed in claim 8, in which the composition is formulated as a shampoo and besides the N-alkoxyalkylamide(s) otherwise also contains from 1 to 45% of anionic surfactant, from 1 to 55% of water, and:
- from 0.01 to 10% of foam-booster; and/or
- from 0.01 to 10% of thickener; and/or
- from 0.001 to 1% of preservative; and/or
- from 0.001 to 1% of chelating agent.

10. Use as claimed in claim 8, in which the composition is formulated as a hair-conditioner and besides the N-alkoxyalkylamide(s) also contains from 10 to 95% of water, from 1.0 to 10% of cationic polymer, from 0.1 to 10% of conditioning agent and:
- from 0.01 to 5% of emulsifier; and/or
- from 0.01 to 5% of thickener; and/or
- from 0.001 to 1% of acidulant.

11. Use as claimed in claim 8, in which the composition is formulated as a hair-spray and besides the N-alkoyxalkylamide(s) also contains from 0.01 to 10% of resin, from 0.01 to 5% of neutralizer, from 0.01 to 5% of plasticizer and from 15 to 40% of solvent.

12. Use as claimed in claim 11, in which the composition in the form of a hair-spray contains from 0.05 to 10% of the N-alkoxyalkylamide(s).

13. Use as claimed in claim 11 or claim 12, in which the hair-spray composition includes from 15 to 60% of propellant.

14. Use as claimed in claim 8, in which the composition is formulated as a mousse and besides the N-alkoxyalkylamide(s) also contains from 0.01 to 10% of hair-fixative, from 0.1 to 5% of conditioner, from 0.01 to 5% of foaming agent and from 1 to 50% of solvent.

15. Use as claimed in claim 14, in which the composition in the form of a mousse contains from 0.1 to 10% of hair-fixative and from 0.05 to 5% of foaming agent.

## Patentansprüche

1. Verwendung eines oder mehrerer N-Alkoxyalkylamid(e) der allgemeinen Formel 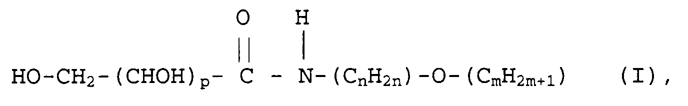
worin p eine positive ganze Zahl von 1 bis 4 ist,
(CₙH₂ₙ) eine gerad- oder verzweigtkettige Alkylenbindungsgruppe darstellt, in der n eine positive ganze Zahl von 1 bis 6 ist, und
(CₘH₂ₘ₊₁) einen gerad- oder verzweigtkettigen Alkylrest darstellt, in dem m eine positive ganze Zahl von 1 bis 6 ist,
bei der Herstellung von Haarpflegezusammensetzungen, die den Zustand des Haares verbessern.

2. Verwendung nach Anspruch 1, wobei das N-Alkoxyalkylamid jenes darstellt oder einschließt, worin p 1 bis 4 ist, n 1 bis 4 ist und m 1 bis 4 ist.

3. Verwendung nach Anspruch 1 oder Anspruch 2, wobei das N-Alkoxyalkylamid jenes darstellt oder einschließt, worin p 4 ist, n 3 ist und m 1 ist.

4. Verwendung nach einem der vorangehenden Ansprüche, wobei das N-Alkoxyalkylamid N-Methoxypropylgluconamid darstellt oder einschließt.

5. Verwendung nach einem der vorangehenden Ansprüche, wobei die Zusammensetzung so formuliert ist, daß sie 0,001 bis 10% des/der N-Alkoxyalkylamids(e) enthält.

6. Verwendung nach einem der vorangehenden Ansprüche, wobei die Zusammensetzung so formuliert ist, daß sie 0,005 bis 7% des/der N-Alkoxyalkylamids(e) enthält.

7. Verwendung nach einem der vorangehenden Ansprüche, wobei die Zusammensetzung so formuliert ist, daß sie 0,01 bis 5% N-Methoxypropylgluconamid enthält.

8. Verwendung nach einem der vorangehenden Ansprüche, wobei die Haarpflegezusammensetzung als ein Shampoo, Konditionierungsmittel, Haarspray, Mousse oder Gel formuliert ist.

9. Verwendung nach Anspruch 8, wobei die Zusammensetzung als ein Shampoo formuliert ist und neben dem/den N-Alkoxyalkylamid(en) ebenfalls noch 1 bis 45% anionisches Tensid, 1 bis 55% Wasser und:
- 0,01 bis 10% Schaumbremser; und/oder
- 0,01 bis 10% Verdickungsmittel; und/oder
- 0,001 bis 1% Konservierungsmittel; und/oder
- 0,001 bis 1% Chelatbildungsmittel enthält.

10. Verwendung nach Anspruch 8, wobei die Zusammensetzung als Haarkonditionierungsmittel formuliert ist und neben dem/den N-Alkoxyalkylamid(en) ebenfalls 10 bis 95% Wasser, 1,0 bis 10% kationisches Polymer, 0,1 bis 10% Konditionierungsmittel und:
- 0,01 bis 5% Emulgator, und/oder
- 0,01 bis 5% Verdickungsmittel, und/oder
- 0,001 bis 1% Säuerungsmittel enthält.

11. Verwendung nach Anspruch 8, wobei die ZusammenSetzung als Haarspray formuliert ist und neben dem/den N-Alkoxyalkylamid(en) ebenfalls 0,01 bis 10% Harz, 0,01 bis 5% Neutralisierungsmittel, 0,01 bis 5% Weichmacher und 15 bis 40% Lösungsmittel enthält.

12. Verwendung nach Anspruch 11, wobei die Zusammensetzung in Form eines Haarsprays 0,05 bis 10% des/der N-Alkoxyalkylamids(e) enthält.

13. Verwendung nach Anspruch 11 oder Anspruch 12, wobei die Haarsprayzusammensetzung 15 bis 60% Treibmittel einschließt.

14. Verwendung nach Anspruch 8, wobei die Zusammensetzung als ein Mousse formuliert ist und neben dem/den N-Alkoxyalkylamid(en) ebenfalls 0,01 bis 10% Haarfixativ, 0,1 bis 5% Konditionierungsmittel, 0,01 bis 5% Schäumungsmittel und 1 bis 50% Lösungsmittel enthält.

15. Verwendung nach Anspruch 14, wobei die Zusammensetzung in Form eines Mousse 0,1 bis 10% Haarfixativ und 0,05 bis 5% Schäumungsmittel enthält.

## Revendications

1. Utilisation, dans la fabrication de compositions pour les soins des cheveux, qui améliorent l'état des cheveux, d'un ou plusieurs N-alcoxyalkylamide(s) de formule générale : 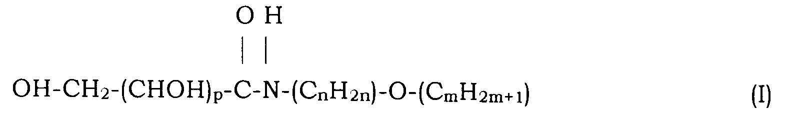
dans laquelle p est un entier positif de 1 à 4,
(CₙH₂ₙ) est un enchaînement alkylène à chaîne droite ou ramifiée dans lequel n est un entier positif de 1 à 6, et
(CₘH₂ₘ₊₁) est un radical alkyle à chaîne droite ou ramifiée dans lequel m est un entier positif de 1 à 6.

2. Utilisation selon la revendication 1, dans laquelle le N-alcoxyalkylamide est ou comprend un N-alcoxyalkylamide dans lequel p vaut de 1 à 4, n vaut de 1 à 4 et m vaut de 1 à 4.

3. Utilisation selon la revendication 1 ou 2, dans laquelle le N-alcoxyalkylamide est ou comprend un N-alcoxyalkylamide dans lequel p vaut 4, n vaut 3 et m vaut 1.

4. Utilisation selon l'une quelconque des revendications précédentes, dans laquelle le N-alcoxyalkylamide est ou comprend le N-méthoxy-propyl-gluconamide.

5. Utilisation selon l'une quelconque des revendications précédentes, dans laquelle la composition est formulée de façon à contenir de 0,001 à 10 % du ou des N-alcoxyalkylamide(s).

6. Utilisation selon l'une quelconque des revendications précédentes, dans laquelle la composition est formulée de façon à contenir de 0,005 à 7 % du ou des N-alcoxyalkylamide(s).

7. Utilisation selon l'une quelconque des revendications précédentes, dans laquelle la composition est formulée de façon à contenir de 0,01 à 5 % de N-méthoxy-propyl-gluconamide.

8. Utilisation selon l'une quelconque des revendications précédentes, dans laquelle la composition pour les soins des cheveux est formulée sous forme d'un shampooing, d'un conditionnant, d'un spray pour cheveux, d'une mousse ou d'un gel.

9. Utilisation selon la revendication 8, dans laquelle la composition est formulée sous forme d'un shampooing et, outre le ou les N-alcoxyalkylamide(s), contient aussi par ailleurs de 1 à 45 % d'un tensioactif anionique, de 1 à 55 % d'eau, et
- de 0,01 à 10 % d'un renforçateur de mousse, et/ou
- de 0,01 à 10 % d'un épaississant, et/ou
- de 0,001 à 1 % d'un conservateur, et/ou
- de 0,001 à 1 % d'un agent chélatant.

10. Utilisation selon la revendication 8, dans laquelle la composition est formulée sous forme d'un conditionnant pour cheveux et, outre le ou les N-alcoxyalkylamide(s), contient aussi de 10 à 95 % d'eau, de 1,0 à 10 % d'un polymère cationique, de 0,1 à 10 % d'un agent conditionnant, et
- de 0,01 à 5 % d'un émulsifiant, et/ou
- de 0,01 à 5 % d'un épaississant, et/ou
- de 0,001 à 1 % d'un acidulant.

11. Utilisation selon la revendication 8, dans laquelle la composition est formulée sous forme d'un spray pour cheveux, et, outre le ou les N-alcoxyalkylamide(s), contient de 0,01 à 10 % d'une résine, de 0,01 à 5 % d'un neutralisant, de 0,01 à 5% d'un plastifiant et de 15 à 40 % d'un solvant.

12. Utilisation selon la revendication 11, dans laquelle la composition sous forme d'un spray pour cheveux contient de 0,05 à 10 % du ou des N-alcoxyalkylamide(s).

13. Utilisation selon la revendication 11 ou 12, dans laquelle la composition de spray pour cheveux contient de 15 à 60 % d'un propulseur.

14. Utilisation selon la revendication 8, dans laquelle la composition est formulée sous forme d'une mousse et, outre le ou les N-alcoxyalkylamide(s), contient aussi de 0,01 à 10 % d'un fixatif pour cheveux, de 0,1 à 5 % d'un conditionnant, de 0,01 à 5 % d'un agent moussant et de 1 à 50 % d'un solvant.

15. Utilisation selon la revendication 14, dans laquelle la composition sous forme d'une mousse contient de 0,1 à 10 % d'un fixatif pour cheveux et de 0,05 à 5 % d'un agent moussant.
